# EUROPEAN PATENT APPLICATION

(11) **EP 4 606 798 A1**
(43) Date of publication of application: **27.08.2025**
(21) Application number: 23867204.2
(22) Date of filing: 23.08.2023
(51) Int. Cl.: C07D 417/12, A61K 31/496, A61P 15/10

(54) **BENZOISOTHIAZOLE COMPOUND, AND PHARMACEUTICAL COMPOSITION AND USE THEREOF**

(30) Priority: 19.09.2022 CN 202211135525
(71) Applicant: Picomole Therapeutics (Liaoning) Co., Ltd., Shenyang, Liaoning 110168 (CN)
(72) Inventor: WANG, Peng, Shenyang, Liaoning 110168 (CN)
(74) Representative: Kirkpatrick
(86) International application number: PCT/CN2023/114338
(87) International publication number: WO 2024/060911

(57) **Abstract**

Provided are a benzisothiazole compound and application thereof, and a pharmaceutical composition and application thereof, which relate to the technical field of medications, and specifically relate to a 2H-benzotriazole-substituted benzisothiazole compound having a novel structure shown in a formula (I), as well as a pharmaceutical composition including the compound, and use of the pharmaceutical composition for the manufacture of a medicament for treatment and/or prevention of premature ejaculation. The anti-premature ejaculation activity of the compound is significantly higher than that of a marketed drug, namely dapoxetine, as studied by *in vivo* activity tests.

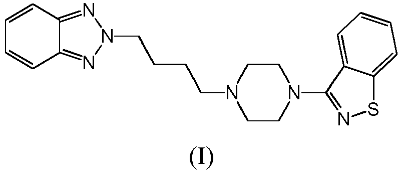

## Description

### FIELD OF THE INVENTION

The present invention relates to the technical field of medications, and in particular relates to a benzisothiazole compound and application thereof, and a pharmaceutical composition and application thereof.

### THE PRIOR ARTS

Premature ejaculation is one of the most commonly reported male sexual dysfunctions. The incidence of the premature ejaculation in adult males in the world is 20%-30%, which seriously affects husband-wife affection and family harmony. In 2014, The International Society for Sexual Medicine (ISSM) proposed the first comprehensive definition of primary premature ejaculation based on evidence. At present, the main medications used in clinical application are dapoxetine, local anesthetics, etc., which has opened up a new way for pharmacological treatment of the premature ejaculation. However, these medications have some problems, such as poor efficacy, many adverse reactions and high withdrawal rate. The dapoxetine is currently the only medication on the market worldwide for the treatment of premature ejaculation, has common adverse reactions in clinical practice, such as nausea, diarrhea, headache and insomnia, and also has extremely low possibility of serious adverse reactions, such as fainting. The dapoxetine has a high clinical withdrawal rate, with the cumulative withdrawal rate increasing over time. The local anesthetics can delay ejaculation time by reducing the sensitivity of a glans penis, but common adverse reactions include erectile dysfunction, which causes poor sexual satisfaction.

Clinicians often perform off-label use of 5-HT selective serotonin reuptake inhibitor (SSRIs) medications, but patients cannot stop the medications abruptly and need to taper off the medications over 2-3 months to prevent SSRI withdrawal syndromes, thus resulting in limited clinical use and low compliance. After long-term use of psychotropic medications, patients themselves have poor acceptability. Therefore, researching new drugs for treating premature ejaculation has clinical significance.

### SUMMARY OF THE INVENTION

In response to the above problems, the present invention discloses a 2H-benzotriazole-substituted benzisothiazole compound having a novel structure, as well as a pharmaceutical composition comprising the compound, and use of the pharmaceutical composition for the manufacture of a medicament for treatment or prevention of premature ejaculation

The present invention discloses the benzisothiazole compound as shown in a formula (I) and the pharmaceutically acceptable salt thereof, where the salt comprises hydrochloride, hydrobromide, sulfate, trifluoroacetate, methanesulfonate, tartrate, malate, citrate, succinate, etc., and may contain 0.5-3 molecules of crystal water:

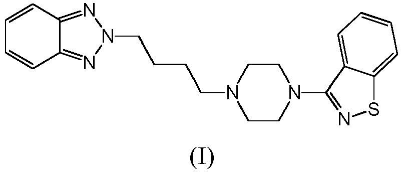

A pharmaceutical composition, including the benzisothiazole compound or the pharmaceutically acceptable salt thereof, and a pharmaceutically acceptable carrier. The carrier refers to a conventional carrier in the field of pharmacy, including: a diluent, and an excipient such as water; a binder such as a cellulose derivative, gelatin and polyvinylpyrrolidone; a filler such as starch; a disintegrant such as calcium carbonate, and sodium bicarbonate; and a lubricant such as calcium stearate or magnesium stearate. In addition, other adjuvants such as flavoring agents and sweeteners may be added to the pharmaceutical composition. When used for oral administration, the pharmaceutical composition can be prepared to form conventional solid formulations, such as tablets, powder, oral soluble film formulations or capsules; when used for external use, the pharmaceutical composition is prepared to form a spray, a liniment and a paste; and when used for injection, the pharmaceutical composition can be prepared to form an injection solution.

Various dosage forms of the pharmaceutical composition of the present invention are prepared according to conventional methods in the field, where the content of active ingredients is 0.1%-99.5% (weight ratio).

Use of the benzisothiazole compound, or the pharmaceutically acceptable salt thereof, or the pharmaceutical composition including the compound for the manufacture of a medicament for treatment and/or prevention of premature ejaculation.

The present invention has the following beneficial effects:
the compound of the present invention has remarkable anti-premature ejaculation effects, and the product has remarkable therapeutic characteristics and substantial technical progress compared with dapoxetine, the only medication approved for marketing.

The compound or the pharmaceutically acceptable salt thereof of the present invention are a 2H-benzotriazole-substituted benzisothiazole compound having a new structure. The anti-premature ejaculation activity of the compound is significantly higher than that of a marketed drug, namely dapoxetine, as studied by *in vivo* activity tests.

### BRIEF DESCRIPTION OF DRAWINGS

FIG. 1: a histogram of total ejaculation number in Wistar rats within 30 min after PCA induction, One-way ANOVA, Dunnett post hoc, ***P < 0.001, and compared with a PE model group, N=5.
FIG. 2: a histogram of the initial ejaculation latency in Wistar rats within 30 min after PCA induction, One-way ANOVA, Dunnett post hoc, *P < 0.05, **P < 0.01, ***P < 0.001, and compared with a PE model group, N=5.
FIG. 3: a histogram of the number of seminal vesicle contractions in Wistar rats within 30 min after PCA induction, One-way ANOVA, Dunnett post hoc, ***P < 0.001, and compared with a PE model group, N=5.
FIG. 4: a histogram of an initial seminal vesicle contractive latency in Wistar rats within 30 min after PCA induction, One-way ANOVA, Dunnett post hoc, **P < 0.01, ***P < 0.001, and compared with a PE model group, N=5.
FIG. 5: a histogram of a seminal vesicle basal pressure in Wistar rats within 30 min after PCA induction, One-way ANOVA, Dunnett post hoc, ***P < 0.001, and compared with a PE model group, N=5.
FIG. 6: a histogram of a seminal vesicle pressure peak in Wistar rats within 30 min after PCA induction, One-way ANOVA, Dunnett post hoc, ***P < 0.001, and compared with a PE model group, N=5.

### DETAILED DESCRIPTION OF THE PREFERRED EMBODIMENT

Reagents and solvents are purchased from Sigma-Aldrich or Fisher Scientific, and can be used without further purification. All reactions are subjected to thin-layer chromatography (silica gel GF-254 thin-layer plate) and LC-MS monitoring. Purification by column chromatography is performed by using 300-400 mesh silica gel (Qingdao Ocean Chemical Co., LTD.). ¹H and ¹³C NMR spectra are recorded in a Bruker AV-400 nuclear magnetic resonance spectrometer for determination, and TMS is used as an internal standard. LC-MS analysis is used for determining that the purity of the compound is greater than 95%, and is used for recording the MS spectra of the compound. LC-MS analysis is performed through Shimadzu LCMS-2020.

### Example 1

### (1) Preparation of a key intermediate: 1-(4-chlorobutyl)-2H-benzotriazole

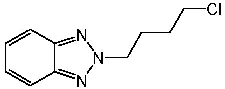

30.0 g (251 mmol) of benzotriazole, 39.3 g (229 mmol) of 1-bromo-4-chlorobutane, 1.85 g (6 mmol) of tetrabutylammonium bromide, and 240 g (20%) of an aqueous sodium hydroxide solution were placed in a 500 mL single-neck bottle, and stirred thoroughly until materials were completely dissolved. The temperature was raised to 60°C, the materials were stirred for reaction for 2 h, and the reaction process was monitored by TLC. After the reaction was completed, extraction was performed with dichloromethane (240 mL ×3), after drying with anhydrous sodium sulfate, a solvent was removed by distillation at reduced pressure, and a light yellow oily liquid (10.23 g) was purified via silica gel column chromatography (yield 19.44%).

### (2) Preparation of a target compound

1.88 g of 3-(1-piperazinyl)-1,2-benzisothiazole, 3.3 g of triethylamine, 1.4 g of potassium iodide, and 15 mL of acetonitrile were added to a 50 mL single-neck bottle, and stirred thoroughly until raw materials were completely dissolved. 1.8 g of 1-(4-chlorobutyl)-2H-benzotriazole was added, temperature was raised to 81°C and reflux reaction was performed for 18 h. After finishing the reaction was detected by TLC, a reaction solution was cooled to room temperature and subjected to suction filtration, and filtrate was distilled under reduced pressure to remove a solvent to obtain yellow oil. The yellow oil was washed with a saturated salt solution, and extracted with dichloromethane, the solvent was removed by distillation under reduced pressure to obtain oil, the oil was dissolved in anhydrous ethanol, a hydrogen chloride ethanol solution was added for adjusting pH to 1, after dropwise addition, a mixture was stirred at room temperature for 1 h, and a solid was precipitated and filtered. A filter cake was recrystallized with anhydrous ethanol to obtain an end product, denoted as A001.

The end product was recrystallized with the anhydrous ethanol to obtain 1.64 g of white powder solid (yield 49%). ¹H NMR (400 MHz, DMSO-*d*₆) δ 10.62 (s, 1H), 8.12 (dd, *J*=11.4, 8.2 Hz, 2H), 7.94 (dd, *J*=6.6, 3.1 Hz, 2H), 7.63-7.54 (m, 1H), 7.50-7.37 (m, 3H), 4.84 (t, *J*=6.8 Hz, 2H), 4.06 (d, *J*=13.6 Hz, 2H), 3.57 (d, *J*=12.0 Hz, 2H), 3.50-3.39 (m, 2H), 3.32-3.19 (m, 4H), 2.19-2.06 (m, 2H), 1.77 (tt, *J*=6.6, 2.5 Hz, 2H).¹³C NMR (100 MHz, DMSO-*d*₆) δ 162.11, 152.06, 143.63, 128.09, 126.89, 126.31, 124.58, 123.96, 121.14, 117.75, 55.27, 54.86, 50.57, 46.36, 26.50, 20.43. LC-MS (ESI) m/z: 393.15 [M+1]⁺.

### Example 2

### In vivo activity study of a compound A001 on a premature ejaculation model

### 2.1 Materials and methods

### 2.1.1 Information of the compound

| Name of the compound | Property | Purity (%) | Source | Batch No. |
|---|---|---|---|---|
| A001 | Powder | 99 | Self-made | NA |
| Dapoxetine hydrochloride | Powder | 100 | Shaanxi Pharmaceutic al Development Center | T0039 |
| p-chloro-alpha-methyl-phenethylaminhydro chloride (PCA) | Powder | 95 | Beijing Lanbote | LMFB022 |

### 2.2 Experimental regimen

### 2.2.1 Information of animals

| Type: | Rat |
|---|---|
| Strain: | Wistar |
| Sex: | Male, chosen at random |
| Body weight: | About 300 g |
| Supplier: | Beijing Vital River Laboratory Animal Technology Co., Ltd. |
| Grade: | SPF |
| Number of animals: | 30 |
| Adaptive phase: | about 7 days |

### 2.2.2 Adaption of animals

Animals were acclimatized with feeding for at least one week after arrival at the animal facility. During this period, animal health status and the occurrence of physiological or behavioral abnormalities were monitored, and all animals displaying anomalies were excluded from the study.

### 2.2.3 Feeding environment

The animal facility environment was controlled at a temperature of 18-26°C, with humidity of 30-70%, and a 12-h light/12-h dark cycle was provided. The 12-hour dark cycle may be temporarily interrupted to align with the study regimen.

### 2.2.4 Food and water

Rat maintenance feeds (provided by Jiangsu Sypharma Bioengineering Co., Ltd.) and reverse osmosis water were consistently available throughout the study.

### 2.2.5 Selection and fasting of animals

The animals used in this study were selected on the basis of their health status and adaptability to cage housing conditions. The animals had ad libitum access to food and water prior to the experiment.

### 2.2.6 Grouping of animals

The animals were subjected to body weight measurement prior to the experiment, and were randomly grouped on the basis of body weight as follows:

| Time for starting grouping | The day before the experiment | | |
|---|---|---|---|
| The number of the animals in each cage | 5 | | |

| Grouping requirements | | The animals were randomly grouped on the basis of body weight. | |
|---|---|---|---|
| Group | The number of animals in each group | Dose of administration | Mode of administration |
| 1-Sham group | 5 | NA | NA |
| 2-PE model group | 5 | Vehicle + PCA 5 mg/kg | p.o. + i.p. |
| 3-Dapoxetine group | 5 | Dapoxetine 2 mg/kg + PCA 5 mg/kg | i.v. + i.p. |
| 4-A001 low-dose group | 5 | A001 0.125 mg/kg + PCA 5 mg/kg | p.o. + i.p. |
| 5-A001 medium-dose group | 5 | A001 0.5mg/kg + PCA 5 mg/kg | p.o. + i.p. |
| 6-A001 high-dose group | 5 | A001 2.0mg/kg + PCA 5 mg/kg | p.o. + i.p. |

| | | | |
|---|---|---|---|
| Note: p.o. indicates oral administration; i.p. indicates intraperitoneal injection; and PCA indicates p-chloro-alpha-methyl-phenethylamine hydrochloride. | | | |

### 2.2.7 Experiment steps:

Male Wistar rats with body weight of about 300 g were subjected to a one-week acclimation period before the experiment began. On the day of the experiment, the animals were anesthetized with intraperitoneal (i.p.) injection of Zoletil 50 at 20 mg/kg combined with xylazine at 8 mg/kg, and then subjected to body temperature maintenance at 37°C using a thermostatically controlled heating pad. The common carotid artery was exposed and cannulated with a PE50 catheter for real-time monitoring of arterial pressure (systolic blood pressure, diastolic blood pressure, and mean arterial pressure). The seminal vesicle and corpus cavernosum muscle of the animals were exposed. Seminal vesicle pressure and corpus cavernosum electromyographic (EMG) stabilized baseline were recorded for 10 min. In the dapoxetine group, the medications were administrated via tail vein intravenous (i.v.) injection; in the to-be-tested compound group, the to-be-tested compound was administrated orally (in the PE model group, the to-be-tested compound was substituted with vehicles); after 1 min, PCA (5 mg/kg) was intraperitoneally (i.p.) injected to induce the PE model; and recording of seminal vesicle pressure profiles and electromyographic (EMG) activity variations in the bulbocavernosus muscle continued to be performed for 30 min.

### Experimental end point:

A. The actual ejaculation number and the first ejaculation latency in animals within 30 min
B. The number of seminal vesicle contractions, the first contractive latency, basal pressure, and peak pressure within 30 min

### 2.2.8 Data analysis

The experimental data was represented by mean ± S.E.M. and statistically analyzed by GraphPad Prism 7.0 software. One-way analysis of variance (One-way ANOVA) was used for comparison among multiple groups, and Dunnett't-test was used for statistical analysis in Post hoc test; and the comparison between two groups was statistically analyzed using Student's t-test. P < 0.05 indicates a significant difference.

### 2.3. Results

**Table 1 Raw data table of various premature ejaculation indexes in Wistar rats within 30 min after PCA induction**

| | Actual ejaculation number | First ejaculation latency (s) | The number of seminal vesicle contractions | First contractive latency (s) | Seminal vesicle basal pressure (mmHg) | Seminal vesicle peak pressure (mmHg) |
|---|---|---|---|---|---|---|
| Sham group | | | | | | |
| **1** | 0 | 1800 | 1 | 1605 | 4.45 | 4.85 |
| **2** | 0 | 1800 | 1 | 1782 | 3.77 | 4.28 |
| **3** | 0 | 1800 | 0 | 1800 | 3.89 | 4.36 |
| **4** | 0 | 1800 | 0 | 1800 | 3.97 | 4.07 |
| **5** | 0 | 1800 | 0 | 1800 | 4.66 | 4.91 |

| PE model group | | | | | | |
|---|---|---|---|---|---|---|
| **6** | 16 | 870 | 19 | 784 | 7.78 | 39.15 |
| **7** | 12 | 843 | 17 | 788 | 8.15 | 37.07 |
| **8** | 25 | 649 | 29 | 605 | 8.49 | 38.44 |
| **9** | 21 | 558 | 26 | 517 | 7.91 | 40.19 |
| **10** | 16 | 915 | 21 | 839 | 8.3 | 40.76 |

| Dapoxetine group | | | | | | |
|---|---|---|---|---|---|---|
| **11** | 1 | 1558 | 1 | 1448 | 5.16 | 6.89 |
| **12** | 0 | 1800 | 1 | 1705 | 4.97 | 8.1 |
| **13** | 0 | 1800 | 0 | 1800 | 5.37 | 6.46 |
| **14** | 1 | 1256 | 1 | 1749 | 5.88 | 6.78 |
| **15** | 2 | 967 | 3 | 866 | 4.96 | 7.03 |

| A001, 0.125 mg/kg group | | | | | | |
|---|---|---|---|---|---|---|
| **16** | 2 | 1456 | 5 | 1278 | 5.79 | 13.46 |
| **17** | 6 | 916 | 9 | 824 | 6.18 | 16.77 |
| **18** | 6 | 985 | 8 | 889 | 6.05 | 20.06 |
| **19** | 2 | 1380 | 4 | 1207 | 4.78 | 11.05 |
| **20** | 1 | 1667 | 3 | 1558 | 3.89 | 9.81 |

| A001, 0.5 mg/kg group | | | | | | |
|---|---|---|---|---|---|---|
| **21** | 2 | 1256 | 3 | 1186 | 5.76 | 11.87 |
| **22** | 2 | 1382 | 4 | 1298 | 5.88 | 10.66 |
| **23** | 2 | 1477 | 4 | 1317 | 5.79 | 9.08 |
| **24** | 0 | 1800 | 0 | 1800 | 3.67 | 4.86 |
| **25** | 0 | 1800 | 0 | 1800 | 3.87 | 5.23 |

| A002, 2 mg/kg group | | | | | | |
|---|---|---|---|---|---|---|
| **26** | 0 | 1800 | 2 | 1568 | 4.05 | 9.67 |
| **27** | 0 | 1800 | 0 | 1800 | 4.17 | 6.04 |
| **28** | 0 | 1800 | 0 | 1800 | 4.86 | 5.18 |
| **29** | 0 | 1800 | 0 | 1800 | 4.18 | 4.98 |
| **30** | 0 | 1800 | 0 | 1800 | 3.88 | 5.05 |

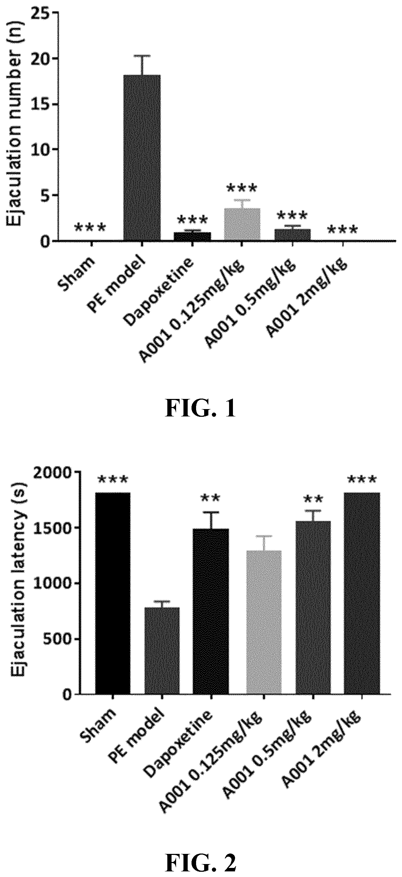

From this study, it was concluded that the ejaculation number in the Wistar rats was basically maintained at about 18 within 30 min after PCA induction, and all dose groups showed highly significant decreases in the ejaculation number compared with the PE model group (all P < 0.05). The low-dose group, the medium-dose group and the high-dose group all had better results than the dapoxetine group (FIG. 1).

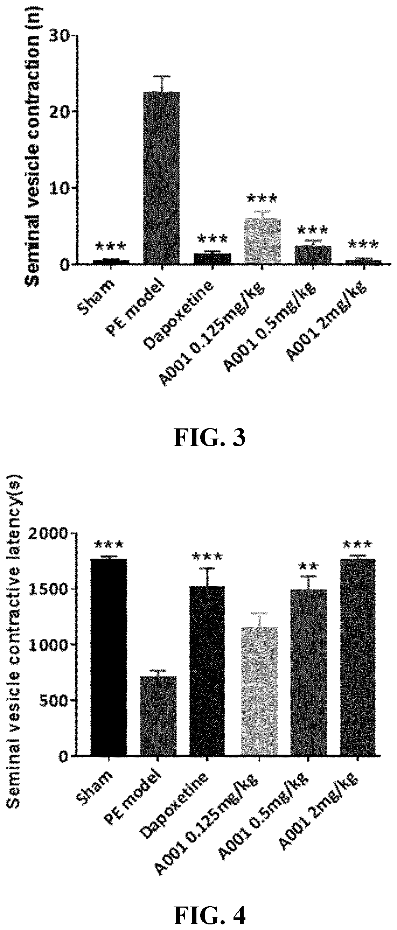

From the results of the initial ejaculation latency, it can be concluded that the low-dose group, the medium-dose group and the high-dose group all showed a significant prolongation in the first ejaculation latency (P < 0.05, P < 0.01, P < 0.001, respectively) compared with the PE model group (FIG. 2). In addition, within 30 min after the PCA induction, the number of seminal vesicle contractions in the rats of the PE model group was about 22.4, the number of seminal vesicle contractions was 6 or below, and all groups showed an extremely significant decrease (all P < 0.05) in the number of seminal vesicle contractions compared with the PE model group (FIG. 3), which was superior to the dapoxetine at the dose of 2 mg/kg.

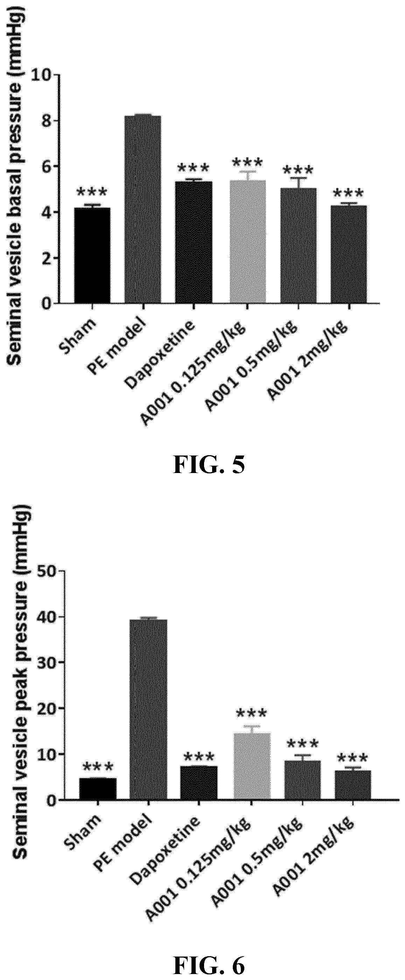

At the same time, from the observation results of the initial seminal vesicle contractive latency in rats, it can be concluded that compared with the PE model group, the Sham group, the dapoxetine group, the medium-dose group and the high-dose group all had a significant increase in the initial seminal vesicle contractive latency (P < 0.01, P < 0.001, respectively) (FIG. 4). In addition, the monitoring data of the seminal vesicle basal pressure showed all groups could effectively reduce the seminal vesicle basal pressure caused by the PCA induction (all P < 0.001) (FIG. 5). Similarly, the monitoring data of the seminal vesicle peak pressure showed all groups could effectively reduce the seminal vesicle peak pressure caused by the PCA induction (all P < 0.001) (FIG. 6).

### 2.4. Conclusions

The potential effects of the tested medication in this patent and the dapoxetine at different doses were monitored through the premature ejaculation model in Wistar rats after PCA induction. From statistical analysis of relevant indexes such as the ejaculation number, the initial ejaculation latency, the seminal vesicle pressure and the contraction of the corpus cavernosum muscle in rats, it was revealed that the dapoxetine and the compound could effectively prolong the ejaculation latency, and significantly decrease the ejaculation number, reduce the seminal vesicle pressure and decrease the contraction number of the corpus cavernosum muscle. Compared with the efficacy of the dapoxetine in treatment of PE, the efficacy of the compound is that oral dose of 2 mg/kg of the compound is superior to the intravenous dose of dapoxetine hydrochloride of 2 mg/kg. With reference to bioavailability of 42% of the dapoxetine, the compound has significantly better anti-premature ejaculation activity than the dapoxetine at the same dose.

## Claims

1. A benzisothiazole compound or a pharmaceutically acceptable salt thereof, wherein the compound has the following structural formula:

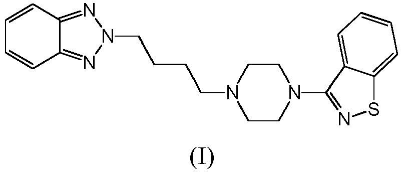

2. A pharmaceutical composition, comprising the benzisothiazole compound or the pharmaceutically acceptable salt thereof according to claim 1, and a pharmaceutically acceptable carrier.

3. The pharmaceutical composition according to claim 2, wherein when used for oral administration, a formulation is selected from tablets, oral soluble film formulations, powder and capsules; when used for external use, the formulation is prepared to form a spray, a liniment and a paste; and when used for injection, the formulation is prepared to form an injection solution.

4. The pharmaceutical composition according to claim 2, wherein the content of active ingredients in various dosage forms of the pharmaceutical composition is 0.1%-99.5%.

5. Use of the benzisothiazole compound or the pharmaceutically acceptable salt thereof according to claim 1 for the manufacture of a medicament for treatment and/or prevention of premature ejaculation.

6. Use of the pharmaceutical composition according to claim 2 for the manufacture of a medicament for treatment and/or prevention of premature ejaculation.
